# FASCICULE DE BREVET EUROPEEN

(11) **EP 2 035 091 B1**
(45) Date de publication et mention de la délivrance du brevet: **26.09.2012**
(21) Numéro de dépôt: 07803980.7
(22) Date de dépôt: 05.07.2007
(51) Int. Cl.: A61N 7/02

(54) **APPAREIL DE THERAPIE A FONCTIONNEMENT SEQUENTIEL**
THERAPIEGERÄT MIT SEQUENZIELLEM BETRIEB
THERAPY APPARATUS WITH SEQUENTIAL FUNCTIONING

(30) Priorité: 05.07.2006 FR 0652813; 05.07.2006 FR 0652816
(43) Date de publication de la demande: 18.03.2009
(73) Titulaire: EDAP S.A., 69120 Vaulx en Velin (FR); INSTITUT NATIONAL DE LA SANTE ET DE LA RECHERCHE MEDICALE (INSERM), 75654 Paris Cédex 13 (FR)
(72) Inventeur: CHAPELON, Jean Yves, F-69100 Villeurbanne (FR); MELO DE LIMA, David, F-69009 Lyon (FR); BLANC, Emmanuel, F-69230 Saint-Genis Laval (FR); CURIEL-RAMIREZ DEL PRADO, Laura, Toronto, ON M4Y1J4 (CA)
(74) Mandataire: Thibault, Jean-Marc
(86) Numéro de dépôt international: PCT/FR2007/051601
(87) Numéro de publication internationale: WO 2008/003910

(56) Documents cités:
- EP-A- 0 104 929
- EP-A- 0 308 644
- EP-A- 0 310 380
- EP-A- 0 367 117
- EP-A- 0 421 290
- WO-A-01/80708

## Description

La présente invention concerne le domaine technique des ultrasons focalisés de haute intensité (HIFU) et elle vise plus précisément le traitement thérapeutique par de tels ultrasons focalisés.

Il est connu que la thérapie par ultrasons focalisés permet de créer des lésions biologiques dans les tissus résultant d'une combinaison des effets thermiques et de l'activité de cavitation acoustique. La forme de ces lésions tissulaires est issue directement du profil du champ ultrasonore appliqué. Ainsi, par exemple dans le cas d'un transducteur de forme sphérique, les lésions tissulaires sont sensiblement elliptiques en étant centrées sur le point de focalisation.

Pour certaines applications, il apparaît le besoin de former des lésions tissulaires en forme de couronne. Par exemple, une lésion tissulaire en forme de couronne peut entourer une tumeur afin de l'isoler, sur un plan vasculaire, et provoquer sa nécrose. De même, une lésion en forme de couronne peut être réalisée à la périphérie d'une veine ou d'une artère à l'endroit de la jonction avec un organe.

L'état de la technique a proposé diverses sondes de traitement par ultrasons focalisés en vue d'obtenir des lésions biologiques en forme de couronne.

Le document US 2006/009 753 décrit un appareil de thérapie dont la sonde de traitement comporte un transducteur cylindrique d'émission d'ondes ultrasonores focalisées à l'aide d'un réflecteur de forme conique. Un tel appareil de thérapie présente une difficulté certaine de réalisation. De plus, la focalisation obtenue avec un réflecteur est moins performante qu'une focalisation directement issue de la géométrie du transducteur.

De même, le document « Local hyperthermia with MR-guided focused ultrasound : Spiral trajectory of the focal point optimized température uniformity in the target région » Journal of magnetic resonance imaging 12 : 571-583 (publié en 2000) décrit un appareil de thérapie comportant un transducteur multiéléments de géométrie sphérique. Ce document décrit la réalisation d'une suite de lésions biologiques suivant une trajectoire en forme de spirale (approchant en ce sens une couronne) entourant le volume à détruire telle qu'une tumeur par exemple. Cette trajectoire est adoptée en vue d'obtenir une dose thermique plus homogène dans le volume à détruire. Cependant, un tel appareil ne permet la formation de lésions biologiques en forme de couronne que par la répétition et la juxtaposition spatiale de lésions élémentaires

La demande de brevet EP 0 421 290 décrit un transducteur de forme sphérique au centre duquel une zone inactive plane est aménagée. Une telle construction a pour effet de modifier le profil de focalisation des ondes ultrasonores jusqu'à obtenir, lorsque le diamètre de la surface plane inactive est suffisamment important, une tâche focale de forme conique dans laquelle le maximum de pression se trouve toujours centré sur l'axe de symétrie du transducteur et non sur une couronne périphérique.

Pour tenter de résoudre ce problème, la demande de brevet EP 0 421 290 propose de découper un transducteur de géométrie sphérique en plusieurs secteurs qui sont écartés les uns des autres ou tournés avec une certaine angulation pour séparer les points de focalisation et les placer à distance de l'axe de symétrie des transducteurs. Il est clair que cette construction mécanique est relativement compliquée à réaliser et difficilement contrôlable. De plus, par construction, le nombre et la position des points focaux sont figés, ce qui limite considérablement les possibilités de traitement. Le document EP 0 367 117 décrit un appareil d'emission à ultrasons ou les transducteurs sont placés sur une surface spherique et ou les émetteurs sont activés de façon simultanée.

D'une manière générale, il est à noter qu'il est toujours possible de synthétiser une tâche focale annulaire à partir d'un transducteur multiéléments de forme sphérique dès lors que chaque élément ultrasonore a une taille inférieure à une demi-longueur d'onde. La longueur d'onde étant très petite par rapport à la taille du transducteur, cette solution ne peut pas être mise en oeuvre en pratique sans entraîner un très grand nombre d'éléments ultrasonores et les problèmes techniques et de coûts associés à ce grand nombre d'éléments ultrasonores.

De façon complémentaire, il doit être considéré que le volume traité dépend de différents paramètres dont, en particulier, la durée d'exposition et l'espacement temporel et spatial entre les tirs ultrasonores. Lorsque ces paramètres sont correctement définis, le volume traité reste restreint à la zone focale de la surface émettrice, ce qui rend cette technique utilisable comme un outil de chirurgie peu invasive.

Pour traiter des volumes plus importants, il est connu de déplacer la zone focale. La première technique consiste à déplacer de façon mécanique l'émetteur ultrasonore entre chaque exposition ultrasonore. Une deuxième technique connue consiste à déplacer de manière électronique la zone focale en excitant les émetteurs avec une loi de retard.

Actuellement, il doit être considéré que les traitements par ultrasons focalisés sont à ce jour principalement réalisés avec des temps de pause entre les expositions ultrasonores pour permettre le refroidissement des tissus et des émetteurs ultrasonores. Il s'ensuit une augmentation relativement importante de la durée de traitement. De plus, l'activité de cavitation et l'échauffement thermique ne sont pas maintenus dans la zone de traitement durant les temps de pause, ce qui atténue l'efficacité du traitement.

Dans l'état de la technique, il a été proposé de réaliser des tirs ultrasonores continus lors desquels les émetteurs ultrasonores et les tissus intermédiaires sont sollicités en permanence créant un risque de destruction des émetteurs ultrasonores et de brûlure des tissus telles que des lésions d'interface ou des brûlures superficielles, en dehors de la zone cible.

L'objet de l'invention vise donc à remédier aux inconvénients de l'état de la technique en proposant un nouvel appareil de thérapie conçu pour permettre de focaliser des ondes ultrasonores selon une couronne et/ou en des volumes focaux distribués selon une couronne, le nombre et la position des volumes focaux pouvant être réglés de manière simple et non limitative en fonction de la lésion tissulaire à obtenir, le fonctionnement de cet appareil permettant de maintenir un échauffement continu des tissus dans la zone de traitement tout en protégeant les tissus situés sur le chemin acoustique en dehors de la zone de traitement.

Pour atteindre un tel objectif, l'objet de l'invention vise à proposer un appareil de thérapie pour le traitement de tissus par l'émission d'ondes ultrasonores focalisées.

Selon l'invention, l'appareil comporte :
- une sonde comportant un transducteur formé par des éléments ultrasonores répartis en plusieurs catégories sur une face d'émission d'ondes ultrasonores focalisées qui est une surface de révolution engendrée par la rotation autour d'un axe de symétrie d'un segment de courbe concave ou convexe de longueur (l) présentant un centre de courbure (C) qui se trouve à une distance (R) non nulle de l'axe de symétrie, les catégories d'éléments ultrasonores définissant des volumes focaux indépendants et sensiblement contigus,
- et un circuit de commande comportant un générateur de signal piloté pour activer indépendamment, alternativement et sensiblement consécutivement les catégories d'émetteurs ultrasonores de manière à assurer une insonification continue selon une couronne, provenant de catégories différentes d'émetteurs ultrasonores.

Selon une variante préférée de réalisation, la surface de révolution est engendrée par un segment d'arc de cercle de longueur donnée, de rayon donné et avec un centre qui se trouve à une distance de l'axe de symétrie avec cette distance ≠ 0.

Selon une autre variante de réalisation, la distance du centre du segment d'arc de cercle à l'axe de symétrie est inférieure au rayon de l'arc de cercle.

Avantageusement, la surface de révolution délimite dans sa région centrale, une ouverture centrée sur l'axe de symétrie et adaptée pour recevoir un transducteur d'imagerie.

Selon une caractéristique avantageuse de l'objet de l'invention, les catégories d'émetteurs ultrasonores correspondent soit à des anneaux concentriques, soit à des secteurs radiaux, soit à des groupes radiaux d'émetteurs ultrasonores.

Selon une première variante de réalisation, les émetteurs ultrasonores sont répartis selon des anneaux concentriques et en ce que le circuit de commande comporte un générateur de signal piloté pour délivrer des signaux pour activer les émetteurs ultrasonores de chacun desdits anneaux concentriques, avec une loi de retard ou de phase afin d'assurer un déplacement de la couronne de focalisation selon l'axe de focalisation.

Selon une autre forme de réalisation, les émetteurs ultrasonores sont répartis en secteurs radiaux et en ce que le circuit de commande comporte un générateur de signal piloté pour délivrer des signaux pour activer les émetteurs ultrasonores de chacun desdits secteurs radiaux, avec une loi de retard ou de phase afin d'assurer un déplacement de la couronne de focalisation.

Selon une variante préférée de réalisation, les émetteurs ultrasonores sont répartis en anneaux concentriques, divisés en secteurs radiaux et le circuit de commande comporte un générateur de signal piloté pour délivrer des signaux pour activer les émetteurs ultrasonores de chacun desdits secteurs radiaux, avec une loi de retard ou de phase afin d'assurer un déplacement de la couronne de focalisation.

Avantageusement, les secteurs radiaux d'émetteurs ultrasonores sont regroupés pour former des groupes radiaux et en ce que le circuit de commande comporte un générateur de signal piloté pour délivrer des signaux pour activer les émetteurs ultrasonores de chacun desdits groupes radiaux.

De préférence, le circuit de commande comporte un générateur de signal piloté pour délivrer pour chaque groupe radial, des signaux pour activer les émetteurs ultrasonores de chacun des groupes radiaux, avec une loi de retard ou de phase.

Avantageusement, le circuit de commande comporte un générateur de signal piloté pour délivrer des signaux pour activer selon une séquence qui varie ou qui se répète cycliquement, une catégorie d'émetteurs ultrasonores correspondant soit à un anneau, un secteur ou un groupe radial d'émetteurs ultrasonores.

De préférence, le circuit de commande comporte un générateur de signal piloté pour délivrer des signaux pour activer des ensembles de catégories d'émetteurs ultrasonores composés soit de catégories distinctes d'émetteurs ultrasonores soit de catégories dont au moins certaines sont communes entre les ensembles.

Un autre objet de l'invention vise à diminuer les câbles de connexions des émetteurs ultrasonores. A cet effet, les groupes radiaux forment des ensembles identiques d'émetteurs ultrasonores et le circuit de commande est relié aux émetteurs ultrasonores par l'intermédiaire de câbles coaxiaux, dont d'une part, les âmes de tous les émetteurs ultrasonores de même rang dans les différents groupes radiaux sont reliées ensemble et d'autre part, les masses de tous les émetteurs ultrasonores d'un même groupe radial sont reliées entre elles, le signal pour activer les émetteurs ultrasonores étant converti en énergie ultrasonore uniquement par les émetteurs ultrasonores du ou des groupes radiaux dont les masses sont physiquement connectées à la masse du signal d'activation.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est un schéma d'un appareil de thérapie conforme à l'invention.
Les **Figures 2** à **4** sont des demi vues de variantes de réalisation de sondes faisant partie d'un appareil de thérapie conforme à l'invention.
Les **Figures 5** à **7** sont des vues partielles comprenant différentes organisations des émetteurs ultrasonores faisant partie d'une sonde de thérapie.
Les **Figures 8A** à **8C** sont des chronogrammes illustrant un principe d'activation en fonction du temps des émetteurs ultrasonores d'un appareil de thérapie conforme à l'invention.
La **Figure 8D** représente au cours du temps le traitement obtenu dans la zone de traitement par un appareil de thérapie conforme à l'invention.
La **Figure 9** est un schéma illustrant un principe préféré de connexion des émetteurs ultrasonores.

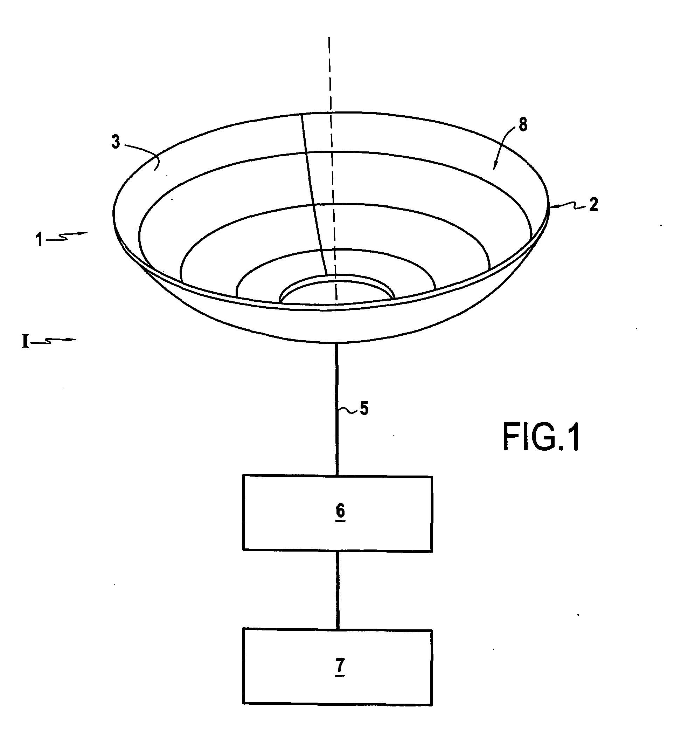

Tel que cela ressort plus précisément de la **Fig. 1****,** l'objet de l'invention concerne un appareil de thérapie **I** au sens général comportant une sonde de thérapie **1** adaptée pour réaliser le traitement de tissus d'un être vivant par l'intermédiaire d'ultrasons focalisés de haute intensité (HIFU). La sonde de thérapie **1** comporte notamment un transducteur **2** comportant un ou plusieurs émetteurs ultrasonores **3** tels que par exemple des éléments piézoélectriques. Ces émetteurs ultrasonores **3** sont reliés par l'intermédiaire de câbles coaxiaux **5** via, un étage amplificateur **6** à un circuit de commande **7** délivrant des signaux pour activer les émetteurs ultrasonores **3**. Le circuit de commande **7** n'est pas décrit plus précisément car sa réalisation fait partie des connaissances techniques de l'homme du métier. Ce circuit de commande **7** comporte ainsi classiquement un générateur de signal piloté qui est relié aux émetteurs ultrasonores par l'intermédiaire de l'étage amplificateur **6.**

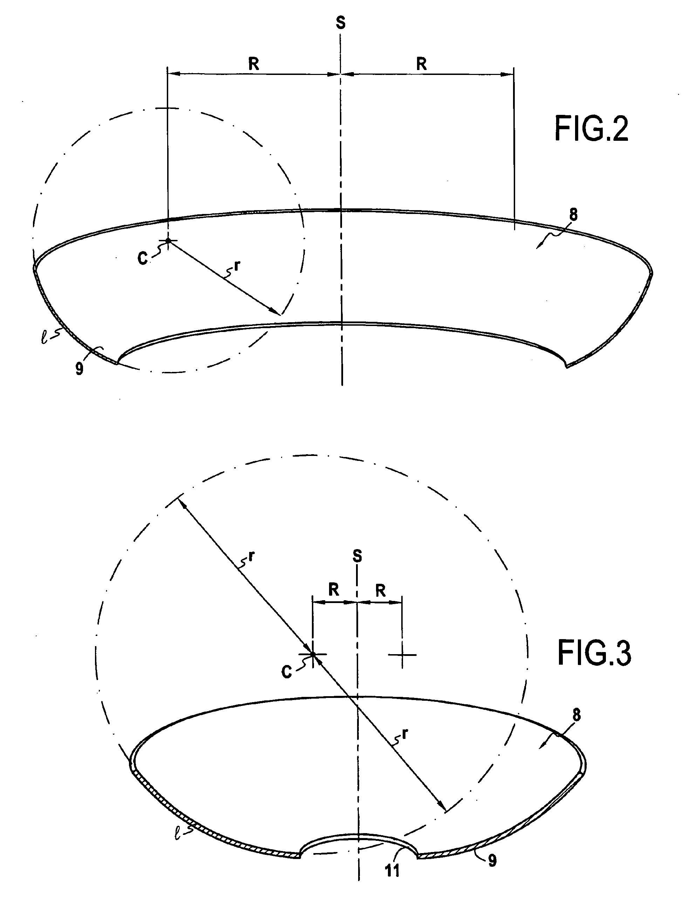

Le transducteur **2** présente une face **8** d'émission d'ondes ultrasonores focalisées. Selon un exemple préféré de réalisation illustré plus particulièrement à la **Fig. 2****,** cette face d'émission **8** est une surface de révolution engendrée par la rotation autour d'un axe de symétrie **S** d'un segment de courbe concave ou convexe **9,** de longueur **l** et présentant un centre de courbure **C** qui se trouve à une distance **R** de l'axe de symétrie **S,** avec **R** différent de zéro.

Selon une première variante préférée de réalisation illustrée à la **Fig. 2****,** la surface de révolution **9** est engendrée par un segment d'arc de cercle de longueur **l,** de rayon **r** et avec un centre **c** qui se trouve à une distance **R** de l'axe de symétrie **S,** avec **R** différent de zéro. La géométrie de cette surface de révolution **9** peut être considérée comme issue d'une géométrie torique.

Selon une variante préférée de réalisation illustrée à la **Fig. 3****,** la surface de révolution **9** est engendrée par un segment d'arc de cercle de centre **C,** de longueur **l** et dont la distance **R** du centre **C** à l'axe de symétrie **S** est inférieure au rayon **r.** Il peut être considéré que la géométrie est issue d'une géométrie relative à un tore dit croisé. Par exemple, la longueur **l** du segment d'arc de cercle présente une valeur inférieure à π r/2.

Tel que cela ressort plus précisément de la **Fig. 3****,** la surface de révolution **9** délimite dans sa région centrale, une ouverture **11** centrée sur l'axe de symétrie **S** et présentant un diamètre par exemple égal à 2.R. Avantageusement, cette ouverture **11** est adaptée pour recevoir un transducteur d'imagerie.

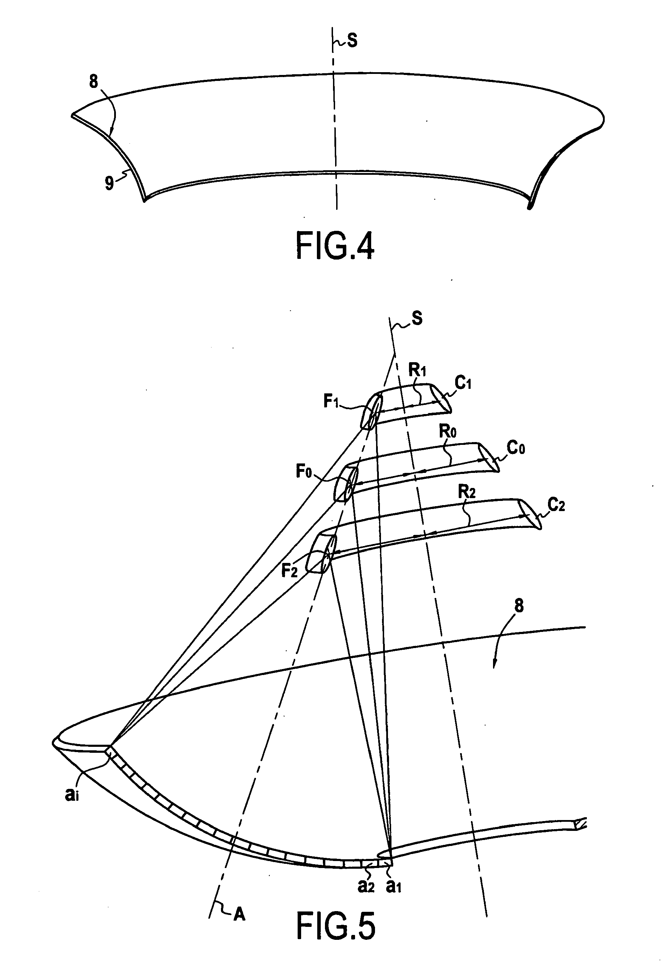

Selon les exemples de réalisation illustrés aux **Fig. 2** et **3****,** la surface de révolution **9** est engendrée par un segment d'un axe de cercle dont la concavité est tournée vers l'axe de symétrie **S.** Bien entendu, la surface de révolution **9** peut être engendrée par un segment d'un arc de cercle dont la convexité est tournée vers l'axe de symétrie **S** comme illustré à la **Fig. 4****.** Dans le même sens, la surface de révolution **9** peut être engendrée par un segment d'une courbe différente d'un arc de cercle. Ainsi, la surface de révolution **9** peut être engendrée par un segment d'une courbe dont la distance **r** entre chaque point du segment de courbe et le centre de courbure **C** présente une variation continue (sans point d'inflexion) tel qu'un segment de courbe elliptique par exemple.

Tel que cela ressort de la description ci-dessus, la face d'émission **8** du transducteur est de géométrie torique. D'une manière générale, la face d'émission **8** du transducteur présente une forme qui est fonction de la géométrie du segment de courbe engendrant la surface émettrice d'ultrasons par rotation autour d'un axe de symétrie, le segment de courbe pouvant présenter des formes diverses.

Selon une autre caractéristique de l'invention, les émetteurs ultrasonores **3** sont organisés sur la face d'émission **8** de manière à être répartis en plusieurs catégories N₁ à N_{M} définissant chacune un point, une tâche, une zone ou d'une manière générale, un volume focal ou un volume de focalisation. Tel que cela ressortira de la suite de la description, les volumes focaux sont indépendants et sensiblement contigus.

La **Fig. 5** illustre un exemple de réalisation dans lequel les émetteurs ultrasonores **3** sont répartis en anneaux concentriques **a₁** à **aᵢ.** Le transducteur **2** selon l'invention est donc formé par un réseau annulaire de i anneaux concentriques constitués chacun d'un émetteur ultrasonore **3.** Ces émetteurs ultrasonores **3** sont activés par des signaux délivrés par le circuit de commande **7** sans loi de retard ou de phase de manière à assurer une focalisation naturelle des ondes ultrasonores selon une couronne **C₀.** Une telle disposition permet ainsi d'assurer une focalisation naturelle selon une couronne située à une distance focale **F₀** par rapport à la face **8** en étant prise sur l'axe de focalisation **A.** Cette couronne de focalisation **C₀** présente un rayon **R₀.**

Selon une variante de réalisation, le circuit de commande **7** délivre des signaux pour activer les émetteurs ultrasonores **3** des anneaux concentriques **a₁** à **aᵢ** avec une loi de retard ou de phase afin d'assurer un déplacement de la couronne de focalisation selon l'axe **A** de focalisation. Le circuit de commande **7** permet ainsi de réaliser une focalisation dynamique électronique de manière à déplacer la couronne focale de sa position naturelle **C₀** jusqu'à une position distale **C₁** et une position proximale **C₂.** Chaque couronne de focalisation **C₁, C₂** est positionnée dans l'espace par la distance focale respectivement **F₁, F₂** et son rayon respectivement **R₁, R₂.** Une telle configuration permet de s'adapter au traitement de tumeurs de tailles différentes et situées à des profondeurs différentes dans les tissus.

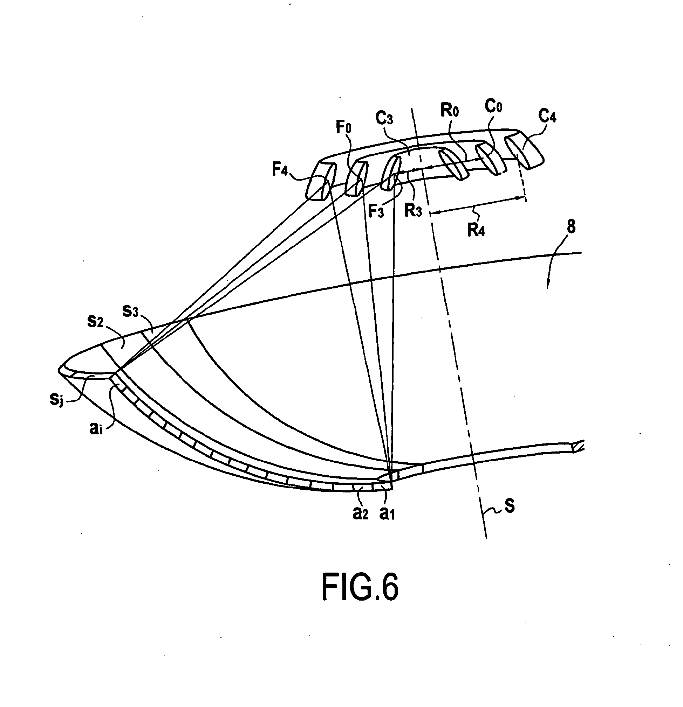

La **Fig. 6** illustre une autre variante de réalisation de répartition géométrique des émetteurs ultrasonores **3.** Dans l'exemple illustré à la **Fig. 6****,** les émetteurs ultrasonores **3** sont répartis en anneaux concentriques **a₁** à **aᵢ** comme illustré à la **Fig. 5** mais se trouvent en plus divisés en secteurs radiaux **s₁** à **sⱼ.** En d'autres termes, la face d'émission **8** du transducteur est découpée par des plans passant par l'axe de symétrie **S** de manière à obtenir une succession de secteurs radiaux. La décomposition du transducteur en émetteurs ultrasonores annulaires et radiaux permet avec des moyens électroniques de focalisation de déplacer la couronne focale de sa position naturelle **C₀** à des positions latérales **C₃** et **C₄.** Chaque couronne focale **C₀, C₃, C₄** est positionnée dans l'espace par la distance focale respectivement **F₀, F₃, F₄** la séparant du transducteur et par son diamètre respectivement **R₀, R₃,** et **R₄.** La couronne de focalisation se trouve ainsi dans un même plan parallèle au plan supérieur du transducteur qui est perpendiculaire à l'axe de symétrie **S.** Il faut noter que la combinaison des moyens de focalisation électronique utilisée pour les configurations décrites dans les **Fig. 5** et **6** permet de modifier l'incidence de ce plan par rapport au plan supérieur du transducteur mais également de réaliser des couronnes déformées selon des surfaces complexes. Cette configuration permet de s'adapter au traitement de tumeurs de tailles différentes.

Il est à noter que la **Fig. 6** illustre une répartition des émetteurs ultrasonores **3** selon des anneaux concentriques **a₁** à **aᵢ,** divisés en secteurs radiaux **s₁** à **sⱼ.** Bien entendu, il peut être prévu que la face d'émission **8** du transducteur comporte une série d'émetteurs ultrasonores **3** seulement divisée en secteurs radiaux **s₁** à **sⱼ.** Avantageusement, les émetteurs ultrasonores **3** de chacun desdits secteurs radiaux sont activés, par le circuit de commande **7,** avec une loi de retard ou de phase afin d'assurer un déplacement de la couronne de focalisation.

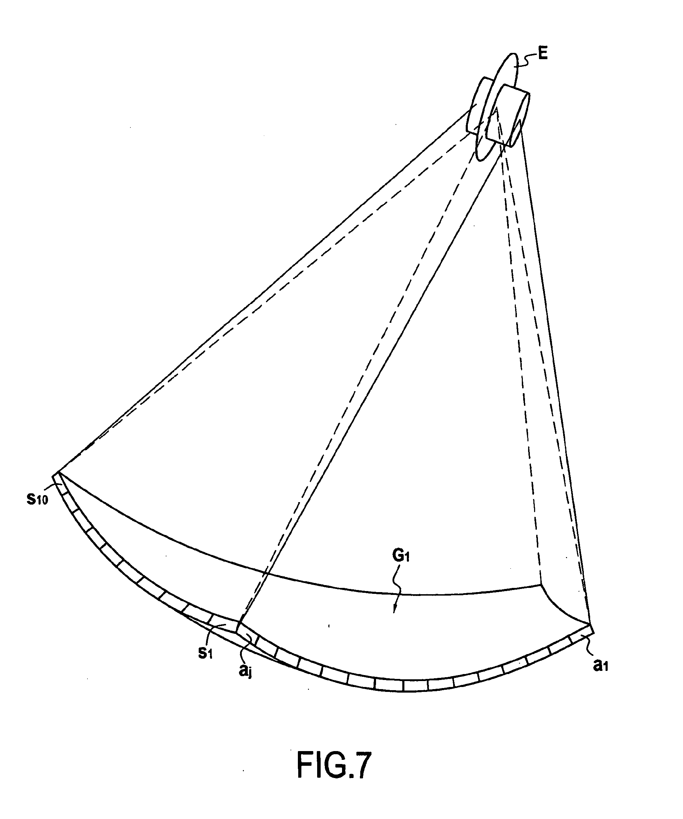

La **Fig. 7** illustre une autre variante de réalisation dans laquelle les secteurs radiaux **s₁** à **sⱼ** d'émetteurs ultrasonores sont regroupés pour former des groupes radiaux **G₁** à **Gₖ.** Il faut entendre par groupe radial le regroupement de différents secteurs radiaux **s₁** à **sⱼ** tel que définis dans l'exemple illustré à la **Fig. 6** de sorte que les émetteurs ultrasonores **3** sont répartis également en anneaux concentriques **a₁** à **aᵢ.** De préférence, les secteurs radiaux **s₁** à **sⱼ** du transducteur sont regroupés de manière identique pour former des groupes successifs identiques **G₁** à **Gₖ.** Dans l'exemple illustré à la **Fig. 7****,** chaque groupe radial **G₁** à **Gₖ** comporte dix secteurs radiaux **s₁** à **sⱼ.** De plus, le transducteur comprend par exemple dix groupes radiaux **G₁** à **G₁₀** comportant tous le même nombre d'émetteurs ultrasonores. Sur la **Fig. 7****,** seul le groupe radial **G₁** est représenté.

Le circuit de commande **7** est adapté pour délivrer des signaux aux émetteurs ultrasonores des groupes radiaux comme décrits ci-dessus. Les émetteurs ultrasonores de chaque groupe radial sont activés de manière à concentrer l'énergie en un volume focal unique. Cette focalisation correspond dans l'exemple illustré à celle qui est obtenue avec un groupe de forme sphérique bien que le groupe soit issu d'une géométrie torique. Il est ainsi possible de concentrer l'énergie distribuée sur une couronne focale en autant de volumes focaux distincts **E** que de groupes d'émetteurs ultrasonores **G₁** à **Gₖ.** Cette concentration permet par exemple de concentrer et de renforcer la nécrose tissulaire en un ou plusieurs points particuliers de la tumeur à traiter. Il peut être envisagé également de former simultanément plusieurs tâches focales ponctuelles réparties sur une couronne focale.

Selon une autre caractéristique de l'objet de l'invention, le générateur de signal du circuit de commande **7** est piloté pour activer indépendamment, alternativement et sensiblement consécutivement les anneaux **a₁** à **aᵢ,** les secteurs radiaux **s₁** à **sⱼ** ou les groupes radiaux d'émetteurs ultrasonores **G₁** à **Gₖ** tels que décrits ci-dessus aux **Fig. 5** et **6** de manière à assurer une insonification continue provenant respectivement d'anneaux, de secteurs ou de groupes radiaux différents. Il doit être compris que l'objet de l'invention vise à activer indépendamment, alternativement et sensiblement consécutivement les émetteurs ultrasonores organisés en plusieurs catégories **N₁** à **Nₘ,** chacune des catégories associant ou regroupant soit un ou plusieurs anneaux concentriques d'émetteurs ultrasonores, soit un ou plusieurs secteurs radiaux d'émetteurs ultrasonores, soit un ou plusieurs groupes radiaux d'émetteurs ultrasonores.

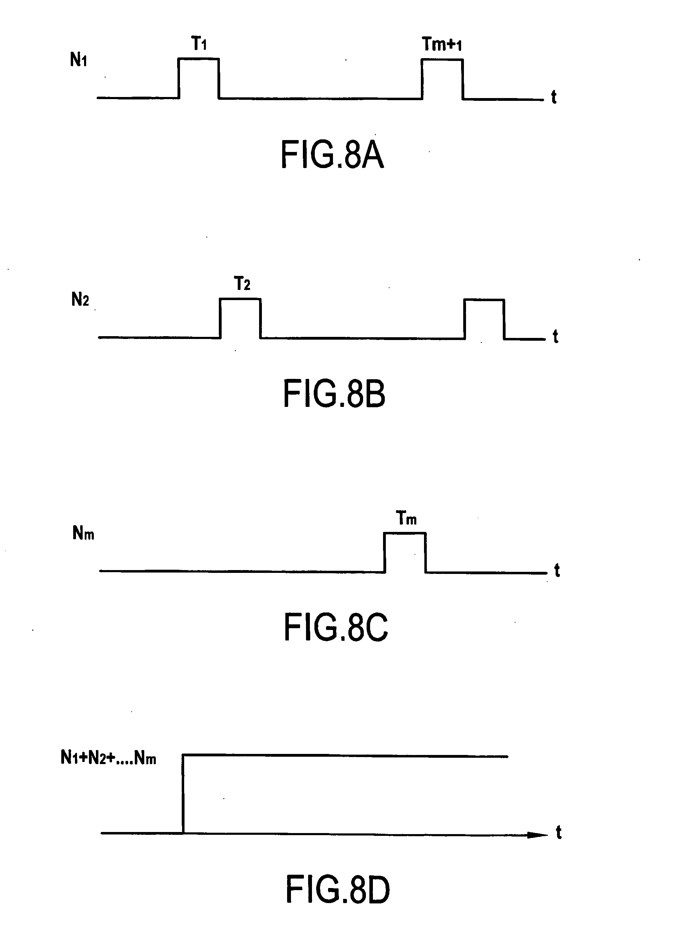

Tel que cela ressort plus précisément des **Fig. 8A** à **8C****,** les émetteurs ultrasonores de la catégorie **N₁** sont activés pour réaliser un tir **T₁.** Au terme de la durée du tir **T₁,** les émetteurs ultrasonores de la catégorie **N₁** ne sont plus activés de manière à laisser refroidir les émetteurs ultrasonores et les tissus intermédiaires situés entre les émetteurs ultrasonores de la catégorie **N₁** et la zone de focalisation. Dès la fin du tir **T₁** de la catégorie **N₁,** les émetteurs ultrasonores de la catégorie **N₂** sont activés de manière à réaliser un tir **T₂** pendant une durée par exemple égale à la durée du premier tir. Le tir **T₂** est interrompu pour laisser refroidir les émetteurs ultrasonores et les tissus intermédiaires situés entre les émetteurs ultrasonores de la catégorie **N₂** et la zone de focalisation. Le circuit de commande **7** poursuit l'activation consécutive des catégories d'émetteurs ultrasonores **N₁** à **Nₘ.** Les catégories d'émetteurs ultrasonores **N₁** à **Nₘ** sont ainsi activées les uns après les autres pour réaliser des tirs respectivement T₁-Tₖₘ₊₁... ; T₂-Tₖₘ₊₂..., avec k = 1, 2... en des volumes de focalisation différents les uns des autres et disposés de manière sensiblement contigüe.

Contrairement aux méthodes de focalisation dynamique, il est à considérer que les émetteurs ultrasonores d'une catégorie sont activés indépendamment et sensiblement consécutivement des émetteurs ultrasonores de l'autre catégorie. Tel que cela ressort de la description qui précède, les catégories d'émetteurs ultrasonores **N₁** à **Nₘ** sont activées alternativement, c'est-à-dire successivement ou les unes à la suite des autres. Comme cela sera décrit plus en détail dans la suite de la description, les catégories d'émetteurs ultrasonores **N₁** à **Nₘ** sont activées selon un ordre ou une séquence déterminée variable ou non.

Avantageusement et idéalement, le tir ultrasonore **T₂** de la catégoriel **N₂** est réalisé immédiatement à la suite du tir ultrasonore **T₁** de la catégorie **N₁** sans retard ou avance. En pratique, il doit être considéré que le circuit de commande **7** active sensiblement consécutivement les catégories des émetteurs ultrasonores de manière à assurer dans la zone de focalisation, une insonification continue. En effet, il s'avère qu'en pratique l'instrumentation mise en oeuvre peut induire une discontinuité ou un recouvrement entre les tirs. Il est considéré qu'un tir **T₂** d'une catégorie **N₂** est sensiblement consécutif à un tir **T₁** d'une catégorie **N₁** si le temps de retard pris entre la fin du tir T₁ et le début du tir **T₂** n'est pas suffisant pour perdre le bénéfice des activités de cavitation et d'échauffement thermique des tirs précédents. De même, deux tirs **T₁** et **T₂** sont considérés sensiblement consécutifs si le temps de recouvrement entre ces tirs est suffisamment faible pour ne pas obtenir une brûlure des tissus.

Tel que cela ressort clairement de la **Fig. 8D****,** la zone de traitement est insonifiée de manière continue tour à tour par une catégorie **N₁** à **Nₘ.** Le traitement ultrasonore est ainsi réalisé de façon continue sans solliciter les mêmes émetteurs ultrasonores. Le temps entre deux tirs ultrasonores est nul ou suffisamment faible pour que les activités de cavitation et d'échauffement thermique soient maintenues au niveau de la zone de traitement. Chaque tir ultrasonore profite alors immédiatement de l'élévation de température et du taux de bulles créé par le tir précédent sans avoir à le reformer entre deux tirs. Cette technique permet de renforcer le volume de chaque lésion associé à chaque catégorie d'émetteurs ultrasonores et/ou de diminuer considérablement le temps de traitement.

La répartition des émetteurs ultrasonores en différentes catégories permet d'appliquer continûment sur un même volume focal la dose thermique nécessaire à la destruction de ce volume à partir de plusieurs catégories distinctes excitées séquentiellement et agissant chacune en un volume focal indépendant et sensiblement contigu.

Il est ainsi possible de traiter des lésions dont le volume est important de façon continue, sans solliciter les mêmes éléments à chaque tir ultrasonore. Dans la mesure où chaque catégorie contient plusieurs émetteurs ultrasonores, il est possible de combiner ce principe de traitement à une focalisation électronique comme décrite ci-dessus. Les émetteurs ultrasonores de chaque catégorie sont alors activés avec une loi de retard ou de phase afin d'assurer un déplacement électronique de la zone de traitement. Il doit être compris que cette loi de retard ou de phase est appliquée uniquement pour les émetteurs ultrasonores appartenant à une même catégorie. En d'autres termes, il n'y a pas de focalisation électronique entre les différentes catégories. Bien entendu, il peut s'envisager d'assurer un déplacement mécanique des émetteurs ultrasonores.

Bien que la zone cible soit chauffée de façon continue, les tissus situés entre les émetteurs ultrasonores et la zone de traitement ne reçoivent des ultrasons que de façon séquentielle, ce qui permet d'obtenir leur refroidissement. En effet, les tissus situés sur le chemin acoustique sont exposés aux ultrasons émis par chaque catégorie uniquement lorsque cette dernière est activée. Par ailleurs, l'échauffement des émetteurs ultrasonores de chaque catégorie est contrôlé du fait de leur activation séquentielle. Le refroidissement des émetteurs ultrasonores d'une catégorie se produit lors de l'activation d'une catégorie suivante.

L'activation des émetteurs ultrasonores peut être réalisée de différentes manières. Ainsi, le circuit de commande **7** peut délivrer des signaux pour activer les catégories d'émetteurs ultrasonores selon une séquence qui se répète cycliquement. Par exemple, les catégories **N₁, N₂, N₃, N₄** sont activées les unes à la suite des autres, et cette séquence d'activation **N₁, N₂, N₃, N₄** est répétée au cours du temps.

Bien entendu, il peut être prévu que la séquence d'activation varie au cours du temps. Par exemple, pendant la première séquence, les catégories **N₁** à **N₄** sont séquentiellement activés dans l'ordre **N₁** à **N₄** alors que lors de la séquence suivante, les catégories sont activées séquentiellement dans l'ordre suivant : **N₁, N₃, N₂, N₄.**

Selon une autre variante possible, le circuit de commande **7** délivre des signaux pour activer des ensembles de catégories d'émetteurs ultrasonores composés de catégories distinctes d'émetteurs ultrasonores. Ainsi, il peut être envisagé une activation séquentielle groupée pour les catégories par exemple **N₁**-**N₂, N₃-N₄.** En d'autres termes, les émetteurs ultrasonores des catégories **N₁** et **N₂** sont activés simultanément puis ceux des catégories **N₃-N₄.** Cette activation séquentielle groupée peut se reproduire cycliquement (**N₁-N₂, N₃-N₄, N₁-N₂, N₃**-**N₄** ...) ou être modifiée sous la forme suivante par exemple (**N₁-N₂, N₃-N₄, N₁-N₄, N₂-N₃** ...).

Selon une autre variante de réalisation, le circuit de commande **7** délivre des signaux pour activer des ensembles de catégories d'émetteurs ultrasonores composés de catégories dont au moins certaines sont communes entre les ensembles. Une telle variante est avantageusement mise en oeuvre par un transducteur comportant un grand nombre de catégories **Ni.** Il peut ainsi être envisagé une activation groupée des catégories **N₁-N₂-N₃-N₄,** puis des catégories **N₄-N₅-N₆-N₇,** puis des groupes **N₇-N₈-N₉-N₁₀,** etc. Selon cet exemple, il apparaît un recouvrement des catégories égal à 25 %. La surface de recouvrement doit être suffisamment faible pour ne pas obtenir lors des tirs consécutifs, une brûlure des tissus situés entre les émetteurs ultrasonores des zones de recouvrement et la zone de focalisation.

L'objet de l'invention trouve des applications particulièrement avantageuses dans tous les traitements par ultrasons focalisés, en particulier le traitement des cancers de la prostate et le traitement des tumeurs du foie.

Selon une autre caractéristique avantageuse, l'objet de l'invention vise à diminuer le nombre de câbles coaxiaux de connexion entre les émetteurs ultrasonores **3** et le circuit de commande **7.** Pour diminuer le nombre de câbles, il est proposé de réaliser une commutation par les masses électriques des émetteurs ultrasonores **3** afin de diminuer de façon importante le nombre de câbles coaxiaux nécessaires ainsi que les besoins en électronique de commande (générateur de signaux et amplificateur de puissance).

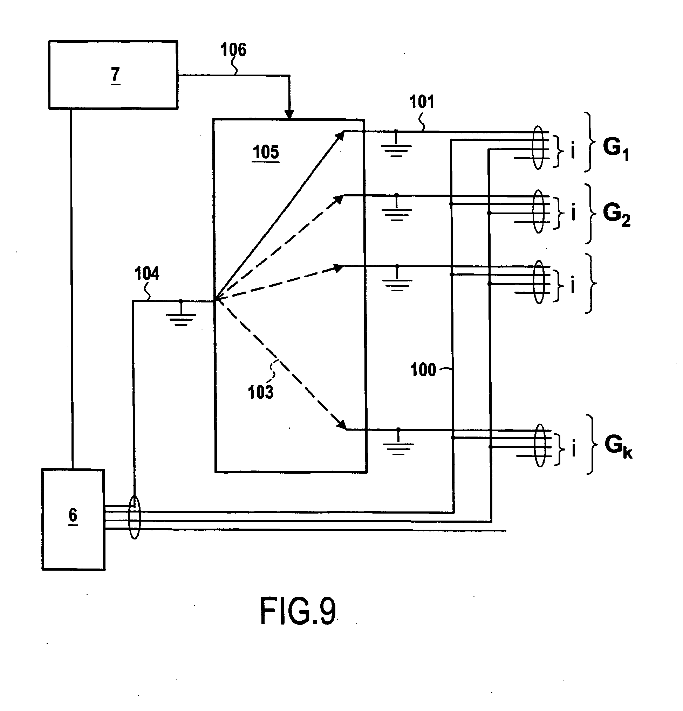

Tel que cela ressort plus précisément de la **Fig. 9****,** le principe est de répartir **les** émetteurs ultrasonores de façon égale et identiques sur les groupes radiaux **G₁** à **G_{K}.** Chaque groupe radial **G₁** à **G_{K}** comporte donc un même nombre **i** d'émetteurs ultrasonores **3.** Tous les émetteurs ultrasonores portant le même numéro dans chaque groupe radial sont identiques et les âmes **100** des câbles coaxiaux de tous ces émetteurs ultrasonores de même rang sont tous connectés ensemble. Les masses **101** des câbles coaxiaux de tous les transducteurs d'un même groupe radial sont reliées ensemble. Il existe ainsi autant de masse que de groupe radial. Les masses **103** des groupes radiaux non utilisés lors d'un tir ultrasonore ne sont pas connectées à la masse **104** du signal d'excitation délivré pour le circuit de commande **7.** Les masses des groupes radiaux activés sont reliées à la masse **104** du signal d'excitation. Ainsi, le signal d'excitation des émetteurs ultrasonores ne sera converti en énergie acoustique uniquement pour les transducteurs du ou des groupes radiaux dont les masses sont physiquement connectés à la masse du signal d'excitation. La commutation d'une masse à l'autre peut se faire au moyen de démultiplexeurs **105** pilotés via une commande **106** par le circuit de commande **7.** A titre d'exemple, pour un transducteur contenant 256 émetteurs ultrasonores répartis de façon égale sur 8 groupes radiaux, il est normalement nécessaire d'utiliser un câble coaxial par transducteur et donc de connecter 257 fils. Par le principe de commutation par les masses, le nombre de câbles est de 40 fils.

L'invention n'est pas limitée aux exemples décrits et représentés car diverses modifications peuvent y être apportées sans sortir de son cadre.

## Revendications

1. Appareil de thérapie pour le traitement de tissus par l'émission d'ondes ultrasonores focalisées, **caractérisé en ce qu'**il comporte :
- une sonde (**1**) comportant un transducteur (**2**) formé par des éléments ultrasonores (**3**) répartis en plusieurs catégories (**N₁** à **N_{M}**) sur une face d'émission d'ondes ultrasonores focalisées (**8**) qui est une surface de révolution engendrée par la rotation autour d'un axe de symétrie (S) d'un segment de courbe concave ou convexe de longueur (**l**) présentant un centre de courbure (**C**) qui se trouve à une distance (**R**) non nulle de l'axe de symétrie (**S**), les catégories d'éléments ultrasonores définissant des volumes focaux indépendants et sensiblement contigus,
- et un circuit de commande (**7**) comportant un générateur de signal piloté pour activer indépendamment, alternativement et sensiblement consécutivement les catégories d'émetteurs ultrasonores de manière à assurer une insonification continue selon une couronne provenant de catégories différentes d'émetteurs ultrasonores.

2. Appareil de thérapie selon la revendication 1, **caractérisé en ce que** la surface de révolution est engendrée par un segment d'arc de cercle de longueur (**l**), de rayon (**r**) et avec un centre (**c**) qui se trouve à une distance (**R**) de l'axe de symétrie avec R ≠ 0.

3. Appareil de thérapie selon la revendication 1 ou 2, **caractérisé en ce que** la distance (**R**) est inférieure au rayon (**r**).

4. Appareil de thérapie selon l'une des revendications 1 à 3, caractérisé en ce la surface de révolution délimite dans sa région centrale, une ouverture (**11**) centrée sur l'axe de symétrie (**S**) et adaptée pour recevoir un transducteur d'imagerie.

5. Appareil de thérapie selon la revendication 1, **caractérisé en ce que** les émetteurs ultrasonores (**3**) sont répartis selon des anneaux concentriques (**a₁** à **aᵢ**) et **en ce que** le circuit de commande (**7**) comporte un générateur de signal piloté pour délivrer des signaux pour activer les émetteurs ultrasonores de chacun desdits anneaux concentriques, avec une loi de retard ou de phase afin d'assurer un déplacement de la couronne de focalisation selon l'axe de focalisation (**A**).

6. Appareil de thérapie selon la revendication 1, **caractérisé en ce que** les émetteurs ultrasonores (**3**) sont répartis en secteurs radiaux (**s₁** à **sⱼ**) et **en ce que** le circuit de commande (**7**) comporte un générateur de signal piloté pour délivrer des signaux pour activer les émetteurs ultrasonores (**3**) de chacun desdits secteurs radiaux, avec une loi de retard ou de phase afin d'assurer un déplacement de la couronne de focalisation.

7. Appareil de thérapie selon la revendication 1, **caractérisé en ce que** les émetteurs ultrasonores (**3**) sont répartis en anneaux concentriques (**a₁** à **aᵢ**), divisés en secteurs radiaux (**s₁** à **sj**) et **en ce que** le circuit de commande (**7**) comporte un générateur de signal piloté pour délivrer des signaux pour activer les émetteurs ultrasonores (**3**) de chacun desdits secteurs radiaux, avec une loi de retard ou de phase afin d'assurer un déplacement de la couronne de focalisation.

8. Appareil de thérapie selon la revendication 7, **caractérisé en ce que** les secteurs radiaux d'émetteurs ultrasonores (**s₁** à **sⱼ**) sont regroupés pour former des groupes radiaux (**G₁** à **Gₖ**) et **en ce que** le circuit de commande (**7**) comporte un générateur de signal piloté pour délivrer des signaux pour activer les émetteurs ultrasonores de chacun desdits groupes radiaux.

9. Appareil de thérapie selon la revendication 8, **caractérisé en ce que** le circuit de commande (**7**) comporte un générateur de signal piloté pour délivrer pour chaque groupe radial (**G₁** à **G_{K}**), des signaux pour activer les émetteurs ultrasonores de chacun des groupes radiaux, avec une loi de retard ou de phase.

10. Appareil de thérapie selon la revendication 1, **caractérisé en ce que** le circuit de commande (**7**) comporte un générateur de signal piloté pour délivrer des signaux pour activer selon une séquence qui varie ou qui se répète cycliquement, une catégorie d'émetteurs ultrasonores.

11. Appareil de thérapie selon la revendication 10, **caractérisé en ce que** le circuit de commande (**7**) comporte un générateur de signal piloté pour délivrer des signaux pour activer des ensembles de catégories d'émetteurs ultrasonores composés soit de catégories distinctes d'émetteurs ultrasonores soit de catégories dont au moins certaines sont communes entre les ensembles.

12. Appareil de thérapie selon l'une des revendications 8 à 11, **caractérisé en ce que** :
- les groupes radiaux (**G₁** à **G_{K}**) forment des ensembles identiques d'émetteurs ultrasonores,
- le circuit de commande (**7**) est relié aux émetteurs ultrasonores par l'intermédiaire de câbles coaxiaux (**5**), dont d'une part, les âmes de tous les émetteurs ultrasonores de même rang dans les différents groupes radiaux sont reliées ensemble et d'autre part, les masses de tous les émetteurs ultrasonores d'un même groupe radial sont reliées entre elles, le signal pour activer les émetteurs ultrasonores étant converti en énergie ultrasonore uniquement par les émetteurs ultrasonores du ou des groupes radiaux dont les masses sont physiquement connectées à la masse du signal d'activation.

## Claims

1. Therapy apparatus for the treatment of tissue by the emission of focused ultrasound waves, **characterised in that** it comprises:
- a probe (1) comprising a transducer (2) formed by ultrasound elements (3) distributed into several categories (N₁ to N_{M}) on an emission face of focused ultrasound waves (8) which is a revolution surface engendered by the rotation about an axis of symmetry (S) of a segment of a curve concave or convex of length (I) having a centre of curve (C) located at a distance (R) from the axis of symmetry (S), the categories of ultrasound elements defining independent and substantially contiguous focal volumes,
- and a control circuit (7) comprising a signal generator controlled to activate independently, alternatively and substantially consecutively the categories of ultrasound emitters so as to ensure continuous insonification according to a crown originating from different categories of ultrasound emitters.

2. Therapy apparatus as claimed in Claim 1, **characterised in that** the revolution surface is engendered by a segment of an arc of a cercle of length (I), of radius (r) and with a centre (c) located at a distance (R) from the axis of symmetry with R ≠ 0.

3. Therapy apparatus as claimed in Claim 1 or 2, **characterised in that** the distance (R) is less than the radius (r) .

4. Therapy apparatus as claimed in any one of Claims 1 to 3, **characterised in that** the revolution surface delimits in its central région an opening (11) centred on the axis of symmetry (S) and adapted to receive an imaging transducer.

5. Therapy apparatus as claimed in Claim 1, **characterised in that** the ultrasound emitters (3) are distributed according to concentric rings (a₁ to aᵢ) and **in that** the control circuit (7) comprises a signal generator controlled to deliver signals to activate the ultrasound emitters of each of said concentric rings, with a delay or phase law for ensuring shifting of the focusing crown according to the focusing axis (A).

6. Therapy apparatus as claimed in Claim 1, **characterised in that** the ultrasound emitters (3) are distributed into radial sectors (s₁ to sⱼ) and **in that** the control circuit (7) comprises a signal generator controlled to deliver signals to activate the ultrasound emitters (3) of each of said radial sectors, with a delay or phase law for ensuring shifting of the focusing crown.

7. Therapy apparatus as claimed in Claim 1, **characterised in that** the ultrasound emitters (3) are distributed into concentric rings (a₁ to aᵢ), divided into radial sectors (s₁ to sⱼ) and **in that** the control circuit (7) comprises a signal generator controlled to deliver signals to activate the ultrasound emitters (3) of each of said radial sectors, with a delay or phase law for ensuring shifting of the focusing crown.

8. Therapy apparatus as claimed in Claim 7, **characterised in that** the radial sectors of ultrasound emitters (s₁ to sⱼ) are combined to form radial groups (G₁ to Gₖ) and **in that** the control circuit (7) comprises a signal generator controlled to deliver signals to activate the ultrasound emitters of each of said radial groups.

9. Therapy apparatus as claimed in Claim 8, **characterised in that** the control circuit (7) comprises a signal generator controlled to deliver for each radial group (G₁ to G_{K}), signals to activate the ultrasound emitters of each of the radial groups, with a delay or phase law.

10. Therapy apparatus as claimed in Claim 1, **characterised in that** the control circuit (7) comprises a signal generator controlled to deliver signals to activate, according to a sequence which varies or which repeats cyclically, a category of ultrasound emitters.

11. Therapy apparatus as claimed in Claim 10, **characterised in that** the control circuit (7) comprises a signal generator controlled to deliver signals to activate assemblies of categories of ultrasound emitters composed either of distinct categories of ultrasound emitters or of categories whereof at least some are common to the assemblies.

12. Therapy apparatus as claimed in any one of Claims 8 to 11, **characterised in that**:
- the radial groups (G₁ to G_{K}) form identical assemblies of ultrasound emitters,
- the control circuit (7) is connected to the ultrasound emitters by means of coaxial cables (5), whereof on the one hand, the cores of all the ultrasound emitters of the same row in the different radial groups are connected together and on the other hand the grounds of all the ultrasound emitters of the same radial group are connected to one another, the signal to activate the ultrasound emitters being converted into ultrasound energy solely by the ultrasound emitters of the radial group(s) whereof the grounds are physically connected to the ground of the activation signal.

## Patentansprüche

1. Therapiegerät für die Behandlung von Geweben durch Aussenden fokussierter Ultraschallwellen, **dadurch gekennzeichnet, daß** es umfaßt:
- eine Sonde (1), die einen Wandler (2) umfaßt, der von Ultraschallelementen (3) gebildet ist, welche in mehreren Kategorien (N₁ bis N_{M}) über eine Fläche zum Aussenden fokussierter Ultraschallwellen (8) verteilt sind, die eine Rotationsfläche ist, welche durch die Rotation -um eine Symmetrieachse (S)- eines konkaven oder konvexen Kurvensegments der Länge (I) mit einem Krümmungsmittelpunkt (C), der in einem Abstand (R) ungleich Null von der Symmetrieachse (S) gelegen ist, erzeugt wird, wobei die Kategorien von Ultraschallelementen unabhängige und im wesentlichen angrenzende Fokalvolumen definieren, und
- einen Steuerkreis (7), der einen gesteuerten Signalgenerator umfaßt, um die Kategorien von Ultraschallsendern unabhängig, abwechselnd und im wesentlichen nacheinander zu aktivieren, so daß eine von unterschiedlichen Kategorien von Ultraschallsendern ausgehende kontinuierliche Beschallung entlang einem Kranz sichergestellt ist.

2. Therapiegerät nach Anspruch 1, **dadurch gekennzeichnet, daß** die Rotationsfläche durch ein Kreisbogensegment mit der Länge (I), dem Radius (r) und mit einem Mittelpunkt (c), der sich in einem Abstand (R) von der Symmetrieachse, mit R ≠ 0, befindet, erzeugt wird.

3. Therapiegerät nach Anspruch 1 oder 2, **dadurch gekennzeichnet, daß** der Abstand (R) kleiner als der Radius (r) ist.

4. Therapiegerät nach einem der Ansprüche 1 bis 3, **dadurch gekennzeichnet, daß** die Rotationsfläche in ihrem mittleren Bereich eine Öffnung (11) begrenzt, die um die Symmetrieachse (S) zentriert und für die Aufnahme eines Bildwandlers ausgelegt ist.

5. Therapiegerät nach Anspruch 1, **dadurch gekennzeichnet, daß** die Ultraschallsender (3) entlang konzentrischer Ringe (a₁ bis aᵢ) aufgeteilt sind und daß der Steuerkreis (7) einen gesteuerten Signalgenerator umfaßt, um Signale zum Aktivieren der Ultraschallsender eines jeden der konzentrischen Ringe mit einem Verzögerungs- oder Phasenverlauf zu liefern, um ein Bewegen des Fokussierkranzes entlang der Fokussierachse (A) sicherzustellen.

6. Therapiegerät nach Anspruch 1, **dadurch gekennzeichnet, daß** die Ultraschallsender (3) in radiale Sektoren (s₁ bis sⱼ) aufgeteilt sind und daß der Steuerkreis (7) einen gesteuerten Signalgenerator umfaßt, um Signale zum Aktivieren der Ultraschallsender (3) eines jeden der radialen Sektoren mit einem Verzögerungs-oder Phasenverlauf zu liefern, um ein Bewegen des Fokussierkranzes sicherzustellen.

7. Therapiegerät nach Anspruch 1, **dadurch gekennzeichnet, daß** die Ultraschallsender (3) in konzentrische Ringe (a₁ bis aᵢ) aufgeteilt sind, welche in radiale Sektoren (s₁ bis sⱼ) unterteilt sind, und daß der Steuerkreis (7) einen gesteuerten Signalgenerator umfaßt, um Signale zum Aktivieren der Ultraschallsender (3) eines jeden der radialen Sektoren mit einem Verzögerungs- oder Phasenverlauf zu liefern, um ein Bewegen des Fokussierkranzes sicherzustellen.

8. Therapiegerät nach Anspruch 7, **dadurch gekennzeichnet, daß** die radialen Ultraschallsendersektoren (s₁ bis sⱼ) gruppiert sind, um radiale Gruppen (G₁ bis Gₖ) zu bilden, und daß der Steuerkreis (7) einen gesteuerten Signalgenerator umfaßt, um Signale zum Aktivieren der Ultraschallsender einer jeden der radialen Gruppen zu liefern.

9. Therapiegerät nach Anspruch 8, **dadurch gekennzeichnet, daß** der Steuerkreis (7) einen gesteuerten Signalgenerator umfaßt, um für jede radiale Gruppe (G₁ bis Gₖ) Signale zu liefern, um die Ultraschallsender einer jeden der radialen Gruppen mit einem Verzögerungs- oder Phasenverlauf zu aktivieren.

10. Therapiegerät nach Anspruch 1, **dadurch gekennzeichnet, daß** der Steuerkreis (7) einen gesteuerten Signalgenerator umfaßt, um Signale zum Aktivieren einer Kategorie von Ultraschallsendern entsprechend einer variierenden oder sich zyklisch wiederholenden Folge zu liefern.

11. Therapiegerät nach Anspruch 10, **dadurch gekennzeichnet, daß** der Steuerkreis (7) einen gesteuerten Signalgenerator umfaßt, um Signale zum Aktivieren von Einheiten von Ultraschallsenderkategorien zu liefern, die entweder aus unterschiedlichen Ultraschallsenderkategorien oder aus Kategorien bestehen, von denen wenigstens einige unter den Einheiten gemein sind.

12. Therapiegerät nach einem der Ansprüche 8 bis 11, **dadurch gekennzeichnet, daß**:
- die radialen Gruppen (G₁ bis Gₖ) identische Einheiten von Ultraschallsendern bilden,
- der Steuerkreis (7) mit den Ultraschallsendern über Koaxialkabel (5) verbunden ist, von denen einerseits die Seelen aller gleichrangigen Ultraschallsender in den verschiedenen radialen Gruppen miteinander verbunden sind und von denen andererseits die Massen aller Ultraschallsender einer gleichen radialen Gruppe untereinander verbunden sind, wobei das Signal zum Aktivieren der Ultraschallsender lediglich durch die Ultraschallsender der radialen Gruppe oder Gruppen in Ultraschallenergie umgewandelt wird, deren Massen mit der Masse des Aktivierungssignals physikalisch verbunden sind.
